# EUROPEAN PATENT APPLICATION

(11) **EP 0 978 257 A1**
(43) Date of publication of application: **09.02.2000**
(21) Application number: 98923238.4
(22) Date of filing: 17.02.1998
(51) Int. Cl.: A61B 5/04

(54) **DEVICE FOR DETECTING PATHOLOGICAL CONDITIONS OF THE MAMMARY GLANDS**

(30) Priority: 30.01.1998 RU 98101088
(71) Applicant: Ioudakov, Sergei Ivanovich, Moscow, 125438 (RU); Dzhmukhadze, Revaz Longinozovich, Moscow, 117296 (RU)
(72) Inventor: Ioudakov, Sergei Ivanovich, Moscow, 125438 (RU); Dzhmukhadze, Revaz Longinozovich, Moscow, 117296 (RU)
(74) Representative: Kiliaridis, Constantin
(86) International application number: RU9800039
(87) International publication number: WO9938435

(57) **Abstract**

This Invention is destined for rapid screening of female population with the purpose of revealing of mammal pathology at earlier stage of disease.

The device consists of two dispersing electrodes (1) and (2) connected to the inputs of differential Amplifier (3); to the output of differential Amplifier (3) the Filter (4), Analog-to-digital Converter (5), block of opto-isolator decoupling (6) and Computer (7) are connect in line.

## Description

### FIELD OF THE INVENTION

This invention is concerned to medicine and intend for screening of female population with the purpose of revealing of mammal pathology at earlier stages of disease and designed for conducting the study in ont-patient and stationary conditions as well as in any suitable premises home conditions.

### BACKGROUND OF THE INVENTION

Known are the devices for radiography of mammal ( SU, **180739**, Cl. A61B 6/04, 1966; SU, **186080**, Cl. A61B 6/02, 1966; SU, **260083**, Cl. A61B 6/02, 1969; SU, **726686**, Cl. A61B 6/02, 1981; US, **4901335**, Cl. A61B 6/04, 1990; EP, **0116345**, A61B 6/02, 1984; EP, **0444869**, A61B 6/00, 1991; EP, **0322260**, A61B 6/00, 1989). All mentioned devices require durable period of time to process the obtained photographs.

Selected as a prototype is the device to diagnose the female person condition using her constant level of potential consisting connected in line electrode of vertex, DC-Amplifier, filter, block of galvanic decoupling made as photon coupled pair, analog-to--digital converter, programming interface and monitor made as computer, in this case, the second input of amplifier is connected to the electrodes of tenors through the switchboard (RU, Cl. A61B 5/05, 1997).

### SUMMARY OF THE INVENTION

The technical result of the invention is simplification of the device and increase of its reliability and the reduction, at the expense of this, of its cost.

The technical result is reached in device for revealing of mammal pathology by the fact that in this device containing two electrodes the dispersing one is connected to the first Differential Amplifier input, the output of which to filter inlet, Analog-to-Digital Converter, block of opto-isolator decoupling and the computer; the second electrode is also dispersing one and is connected to the second Differential Amplifier output, and to the filter outlet the Analog-to-Digital Converter is connected in line as well as Block of opto-isolator decoupling and Computer.

In the opinion of Inventor, the proposed design is in line with the requisites of patentability- novelty (N), inventive level (IS) and industrial applicability (IA).

### BRIEF DESCRIPTION OF THE DRAWINGS

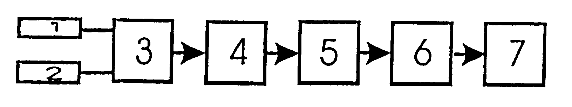

**Fig. 1** depicted the structural electric circuit of the device for revealing of mammal pathology where: 1 and 2 are dispersing electrodes; 3 - differential amplifier; 4- filter; 5 - Analog-to-digital Converter; 6 - Block of opto-isolator decoupling and 7 - Computer and Power Unit.

### PREFERRED EMBODIMENT OF THE INVENTION

In the device, made in accordance with said structural circuit, the power is provided from signal-phase AC of 220V + 10% and frequency of 50Hz-1 Hz (Power Unit is not depicted in the drawing separately) . Power consumption of the device when using it alongside with Computer does not exceed 30 VA. The range of measurement of input value, provided by the device, is in the limits from - 1999mV to +1999mV, in this case the value of less discharge is 1 mV.

As the electrodes 1 and 2 one can use two glass electrodes of liquid 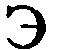 B1-1M3 TYPE.

The differential Amplifier 3 is used for linear amplification to 9V of potential differences measured between two points of person's body. The filter 4 eliminates high frequency components of signal and is required to mate the output signal of differential amplifier 3 to the signal of Analog -to-Digital Converter 5.

Analog -to-Digital Converter 5 convert analog signal to digital one which is required to be set into Computer. Block opto-isolator decoupling 6 is required to prevent ingress of higher voltage from Computer into measuring device. Power supply of the block is provided with A.C. self-contained Power supply Unit of 9V.

The device works as follows:

The dispersing electrodes 1 and 2 are placed on the surface of person skin moistered with physiological solution. In this case the electrodes 1 and 2 can be placed using the combinations as follows:
a) Electrode 1 is placed in the area of right mammila while electrode 2 - in the area of left mamilla;
b) Electrode 2 is placed in the area of right mammila while electrode 1 - in the area of left mamilla;
c) Electrode 1 is placed in the area of right mammila while electrode 2 - in the area of left hand;
d) Electrode 1 is placed in the area of right mammila while electrode 2 - in the area of right hand.

Changing the combinations of electrodes' arrangement the measurement of proper mammal difference of potentials and those of mammal concerning proper potentials of hands is measured. Signal obtained from electrodes 1 and 2 enter inputs of differential amplifier 3 and being further amplified up to 9V the signal enter the input of Analog-to-digital Converter 5 though the filter 4 and is converted to digital signal for subsequent transmission, through the block of opto-isolator decouling 6, to the computer 7, where the information is processed and issued to doctor as a chart and digital data that indicates the deviation from norm and actual values in per cent ratio.

### INDUSTRIAL APPLICABILITY

This device can be used for revealing of mammal pathology of female patients at earlier stages including latent forms of pathology at Mammary Centres while examing female patients providing doctor with information in 5-7 minutes as chart or digital data indicated as per cent ratio of deviation from norm and actual values.

## Claims

1. The device for revealing of mammal pathology containing two electrodes the dispersing one of which is connected to the first Differential Amplifier input, the output of which to filter inlet, Analog-to-Digital Converter, block of opto-isolator decoupling and the computer and distinguished by the fact that the second electrode is also dispersing one and is connected to the second Differential Amplifier output, and to the filter outlet the Analog-to-Digital Converter is connected in line as well as Block of opto-isolator decoupling and Computer.
